# FASCICULE DE BREVET EUROPEEN

(11) **EP 0 631 492 B1**
(45) Date de publication et mention de la délivrance du brevet: **24.09.1997**
(21) Numéro de dépôt: 93904091.1
(22) Date de dépôt: 19.01.1993
(51) Int. Cl.: A61B 17/58

(54) **DISPOSITIF D'OSTEOSYNTHESE RACHIDIENNE**
VORRICHTUNG FUER DIE OSTEOSYNTHESE DER WIRBELSAEULE
SPINAL OSTEOSYNTHESIS DEVICE

(43) Date de publication de la demande: 04.01.1995
(73) Titulaire: JBS, Société Anonyme, F-10000 Troyes (FR)
(72) Inventeur: JEANSON, Jean-François, F-10000 Troyes (FR); CSERNATONY, Zoltan, H-4032 Debrecen (HU)
(74) Mandataire: Viering, Jentschura & Partner
(86) Numéro de dépôt international: FR9300045
(87) Numéro de publication internationale: WO9416635

(56) Documents cités:
- US-A- 4 409 968
- US-A- 4 738 251
- US-A- 4 773 402
- Section PQ, Week 7635, Derwent Publications Ltd., London, GB; Class P31, AN 76- H7053X & SU, A,485 739 (INSTITUT MEDICAL DE CRIMEE) 22 Décembre 1975 voir figure 1 voir abrégé

## Description

L'invention concerne les dispositifs de redressement, d'étaiement et d'ostéosynthèse rachidienne à implants fixés aux vertèbres, reliés entre eux par au moins une barre par l'intermédiaire de moyens de solidarisation amovibles.

Le traitement chirurgical des déformations et des instabilités de la colonne vertébrale a été acquis lors des trois dernières décennies. Cette chirurgie est indiscutablement nécessaire dans certains cas très répandus tel que celui de la scoliose, par exemple. Plusieurs faits ont, cependant, empêché pendant longtemps son évolution et sa généralisation à l'ensemble des cas qui le justifiaient tels que :
- mauvaise appréciation de ces maladies par les chirurgiens et les patients,
- mauvaise interprétation des instabilités et des difformités rachidiennes,
- insuffisance de connaissances en biomécanique,
- manque d'expérience chirurgicale dans cette partie du corps,
- technique de fabrication d'implants sophistiqués encore peu élaborée.

L'événement majeur dans le développement de cette chirurgie a été l'introduction sur le marché en 1961 de l'instrumentation dite de HARRINGTON, qui peut être considérée maintenant comme un peu dépassée, compte-tenu notamment des importants progrès réalisés en biomécanique dans le domaine des prothèses, et que, de nos jours, la chirurgie rachidienne pour les pathologies ci-dessus évoquées, consiste dans l'obtention d'une arthrodèse posto-latérale, a l'exception de quelques méthodes encore peu élaborées telles que les prothèses discales et la ligamentoplastie.

Un dispositif de redressement selon l'état de la technique est aussi montré dans US-A-4 773 402.

La présente invention a pour but de remédier à cette situation. L'invention telle qu'elle se caractérise résout le problème consistant à créer un jeu d'implants pour ostéosynthèse rachidienne en s'inspirant de l'analyse profonde de la formation et du caractère des courbures scoliotiques, ainsi que des échecs et des impasses des instrumentations courantes, plus simple à fabriquer et à implanter, plus efficace quant à la correction, en évitant le recours à des implants intra-canalaires, avec lesquels il soit possible de changer d'orientation chirurgicale, en obtenant la correction des déformations, en sacrifiant la mobilité d'une partie de la colonne à un niveau plus ou moins étendu selon l'importance de la déformation, en recourant à l'ancrage et à la prise des implants dans l'espace costo-transversaire, sur la partie dorsale, afin d'obtenir l'inclinaison, la dérotation, la traction vers l'arrière, ou l'appui vers l'avant des vertèbres instrumentées, ainsi que la compression ou la distraction dans l'axe propre du rachis et l'ancrage limite supérieur et inférieur des montages.

Le dispositif de redressement, d'étaiement ou/et d'ostéosynthèse rachidienne à implants fixés aux vertèbres, reliés entre eux par au moins une barre, par l'intermédiaire d'un moyen de solidarisation amovible, selon l'invention, se caractérise principalement en ce que les implants sont constitués :
- pour les vertèbres dorsales, de deux crochets reliés par un étrier,
- pour les vertèbres lombaires, de deux anneaux reliés par un étrier,
en ce que le moyen de solidarisation amovible des implants aux barres de liaison est constitué d'un crochet à fente basculant comportant, dans le fond de la fente, une cannelure semi-cylindrique de rayon correspondant à celui des barres de liaison et dans le plan médian de sa base, un orifice fileté dans lequel est montée une vis de pression permettant d'obtenir le basculement longitudinal du crochet jusqu'au blocage de l'étrier de l'implant contre la barre de liaison correspondante, et dont le bec est conformé selon la section de l'étrier de liaison des crochets ou des anneaux de l'implant.

Pour obtenir le redressement puis l'étaiement du rachis, les implants à crochets sont :
- à crochets de même sens,
- à crochets inversés, crochet droit vers le haut,
- à crochets inversés, crochet gauche vers le haut.

L'étrier de liaison des crochets ou des anneaux des implants épouse la courbure des vertèbres et comporte des méplats opposés, d'immobilisation en rotation par rapport aux barres, sous l'action du crochet à fente.

Selon un mode de réalisation préférentiel, l'orifice fileté dans lequel se monte la vis de pression de basculement du crochet fendu, est réalisé obliquement selon un angle d'environ 25° vers l'arrière, avec un certain décalage D par rapport à l'aplomb du bec du crochet à fente.

Afin d'accentuer l'action de blocage de l'étrier des implants contre la barre de jonction, la base du crochet à fente est prolongée du côté de l'orifice fileté recevant la vis de basculement et de blocage.

Les avantages obtenus, grâce à cette invention, consistent essentiellement en ceci qu'avec ce dispositif à analogie de l'orthodontie, il est possible d'espérer donner aux patients le soutien et le stimulus nécessaires à la correction des difformités d'un rachis en croissance, sans sacrifice de mobilité, en recourant à des moyens simples, moins traumatisants et plus faciles à implanter que ceux proposés jusqu'ici.

D'autres caractéristiques et avantages apparaîtront dans la description qui va suivre d'un dispositif, selon l'invention, utilisé pour le redressement et l'étaiement d'un rachis scoliosé, donné à titre d'exemple non limitatif au regard des dessins annexés sur lesquels :
- la figure 1 représente une vue de dos d'un rachis scoliosé, implanté mais non encore redressé ni étayé ;
- la figure 2 représente une vue de dos du rachis de la figure 1 après redressement et étaiement ;
- les figures 3a, 3b, et 3c représentent respectivement une vue de face, une vue de côté et une vue de dessus schématiques d'un implant à crochets de même sens ;
- les figures 4a, 4b et 4c représentent respectivement une vue de face, une vue de côté et une vue de dessus schématiques d'un implant à crochets inversés, à crochet droit dirigé vers le haut ;
- les figures 5a, 5b et 5c représentent respectivement une vue de face, une vue de côté et une vue de dessus schématiques d'un implant à crochets inversés, à crochet gauche dirigé vers le haut ;
- les figures 6a, 6b et 6c représentent respectivement une vue de face, une vue de côté et une vue de dessus schématiques d'un implant à anneaux ;
- les figures 7a, 7b, 7c et 7d représentent respectivement une vue en perspective, une vue de dessus, une vue de côté en coupe selon YY, vue suivant F et une vue de côté en coupe selon ZZ, vue suivant G, d'un mode de réalisation d'un implant à crochets inversés, à crochet gauche dirigé vers le haut ;
- les figures 8a, 8b, 8c et 8d représentent respectivement une vue en perspective, une vue de dessus, une vue de côté, en coupe selon VV, vue suivant H et une vue de côté en coupe selon WW, vue suivant l d'un mode de réalisation d'un implant à crochets inversés, à crochet droit dirigé vers le haut ;
- la figure 9 représente une vue en perspective partielle du noeud d'assemblage d'un implant avec une barre d'étaiement par l'intermédiaire d'un crochet à fente ;
- la figure 10 représente une vue de face du crochet à fente avec représentation partielle en trait mixte de la barre d'étaiement et de l'étrier de liaison d'un implant ;
- la figure 11 représente une vue de côté en coupe longitudinale selon le plan XX indiqué sur la figure 10 du crochet à fente en position débloquée avec indication en trait mixte de la barre d'étaiement et de l'étrier de liaison d'un implant ;
- la figure 12 représente le crochet à fente de la figure 9 en position bloquée sur la barre d'étaiement et l'étrier de liaison d'un implant représenté partiellement en trait mixte;
- la figure 13 représente une vue de dessus du crochet à fente de la figure 9 en position bloquée sur une barre d'étaiement et l'étrier d'un implant représentés en trait mixte.

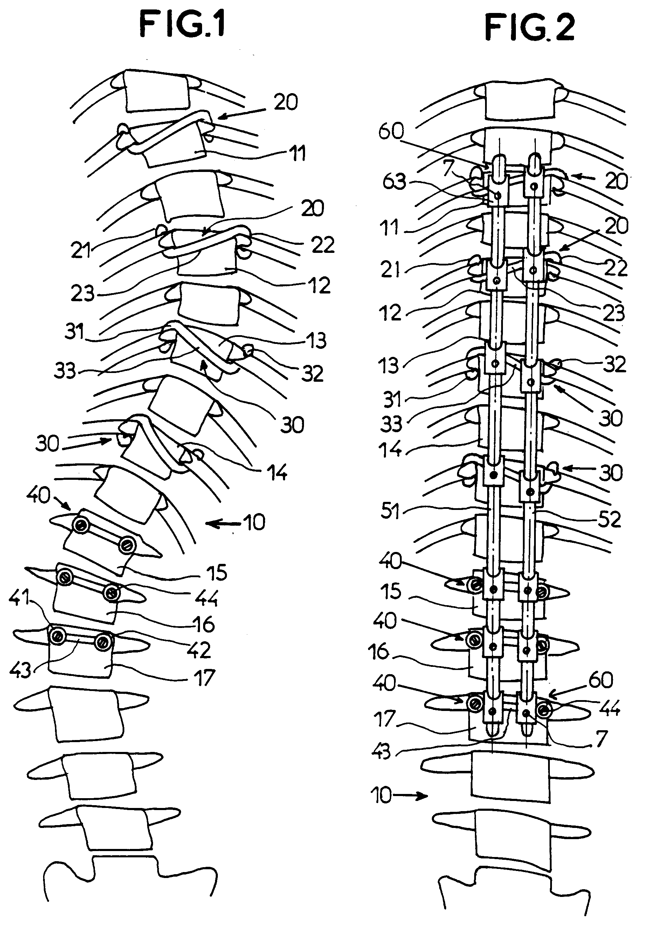

Les figures représentent un rachis 10 dont les vertèbres repérées 11 à 17 ont été munies : pour les vertèbres 11 et 12 d'un implant 20 à crochets 21 et 22, reliés par un étrier 23 à méplats 231 et 232, dont le crochet gauche 21 est dirigé vers le haut et, pour les vertèbres 13 et 14, d'un implant 30 à crochets 31 et 32 reliés par un étrier 33 à méplats 331 et 332 dont le crochet droit 32 est dirigé vers le haut, alors que les vertèbres lombaires 15, 16, 17 ont été munies chacune d'un implant 40 à anneaux 41 et 42 reliés par un étrier 43, fixés par des vis 44 ; ledit rachis 10 ayant été ensuite redressé et étayé, figure 2, par l'intermédiaire de barres 51 et 52, reliées aux étriers 23, 33, 43 des implants 20, 30, 40 par l'intermédiaire de crochets 60 à fente 61 à bec 62 et à talon 63 muni d'une cannelure longitudinale semi-cylindrique 630 côté crochet et d'un orifice fileté 632 recevant une vis 7 de basculement et de blocage.

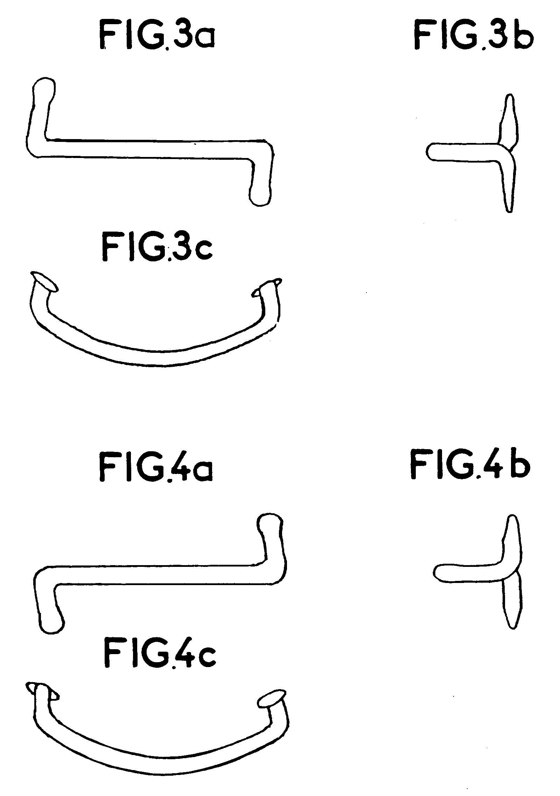

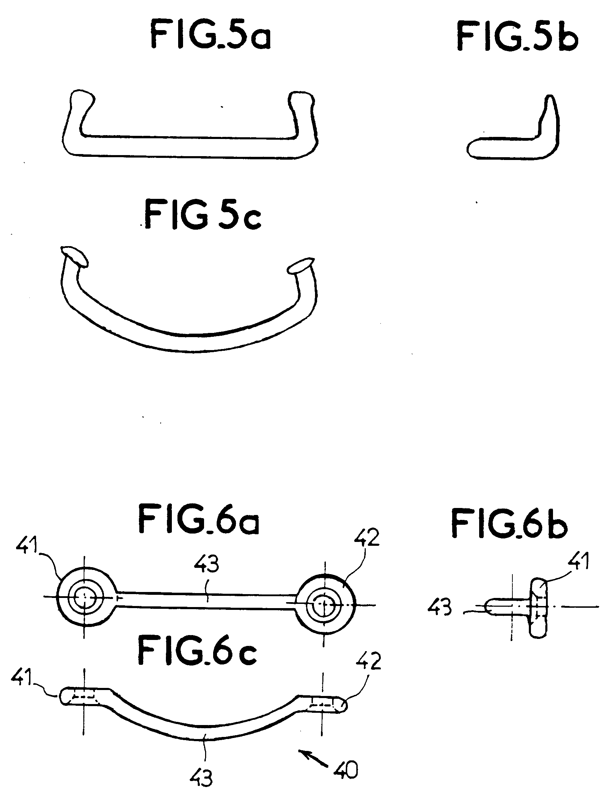

En examinant plus particulièrement les figures 1 à 6c donnant une représentation schématique du dispositif, on remarque qu'il est possible, compte-tenu de la variété de forme des implants proposés, d'obtenir conjointement et selon la déformation du rachis 10, l'inclinaison, la dérotation, la traction vers l'arrière ou l'appui vers l'avant des vertèbres instrumentées, ainsi que, si nécessaire, la compression ou la distraction, dans l'axe propre du rachis, ainsi que l'ancrage limite supérieur et inférieur des montages.

Les implants à crochets fabriqués en deux tailles suffisent pour couvrir toutes les tailles en recourant, si nécessaire à un cintrage à la demande de l'étrier reliant les crochets des implants.

La forme curviligne de ces implants, avec concavité vers l'avant, permet de poser les deux barres 51 et 52 devant, sans que ce soit une obligation, avec la garantie d'obtenir une greffe osseuse continue, sans empreinte de crochet à chaque niveau ; la connexion des implants aux barres étant obtenue par l'intermédiaire des crochets 60.

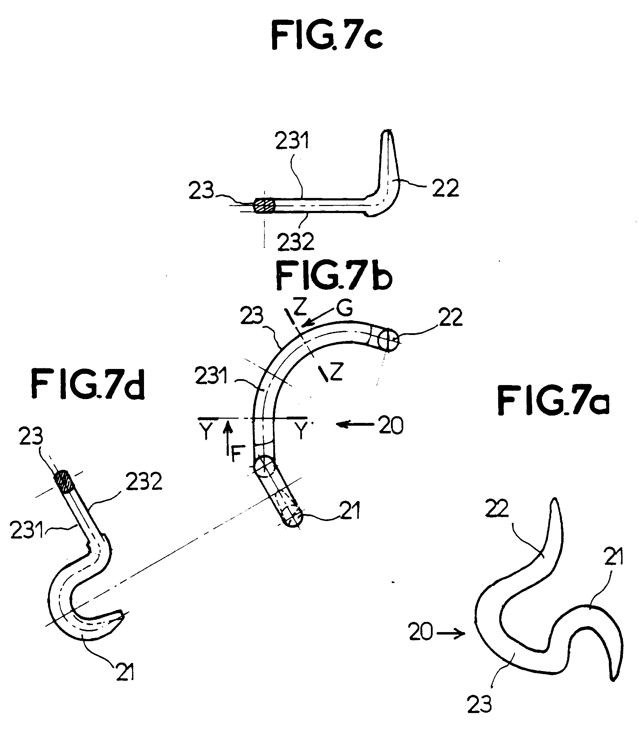

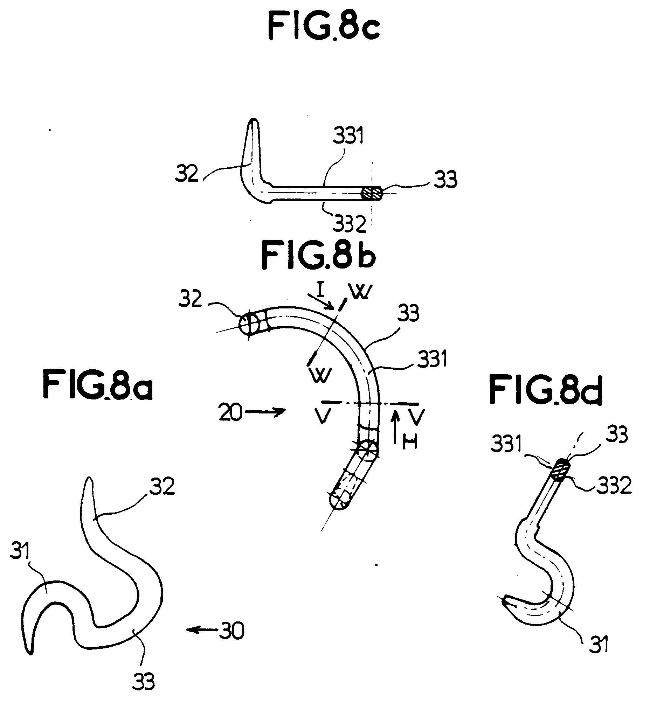

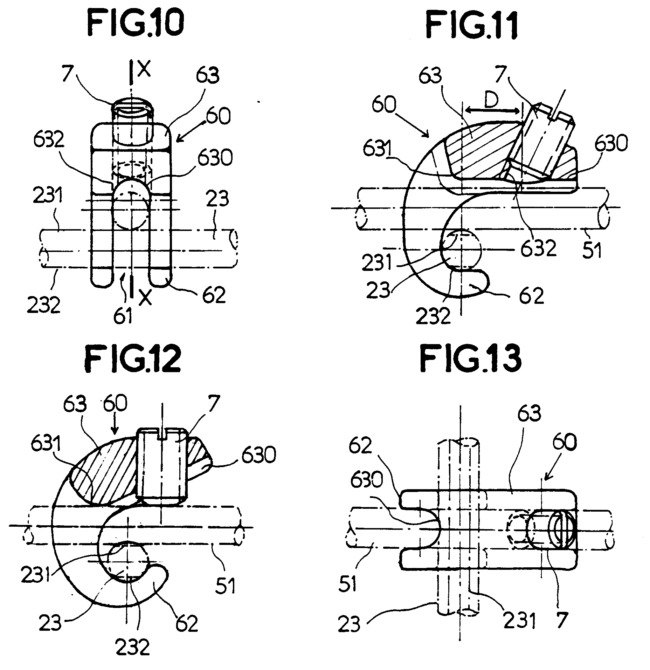

En se rapportant aux figures 7a à 13 représentant un mode de réalisation pratique des principaux éléments du dispositif selon l'invention, on remarque tout d'abord, en examinant les figures 7a à 7d, correspondant à un implant 20 à crochets 21 et 22 inversés, avec crochet gauche 21 dirigé vers le haut puis les figures 8a à 8d correspondant à un implant 30 à crochets 31 et 32 inversés, avec crochet droit 32 dirigé vers le haut, que compte-tenu de la forme particulière de chacun des crochets 21, 22 et 31, 32, il est aisé de faire passer tout d'abord le crochet supérieur 21, 32 derrière la côte gauche ou droite correspondante, puis d'encastrer le bord supérieur de la côte opposée dans l'échancrure de l'autre crochet 22, 31.

On remarque que les méplats 231, 232 et 331,332 réalisés sur les étriers 23, 33 permettent le blocage des implants dans cette position en prenant appui, lors de la solidarisation avec les barres 51, 52 contre une génératrice de chacune des dites barres et contre le bord interne des becs des crochets 60 à fente 61 assurant cette solidarisation.

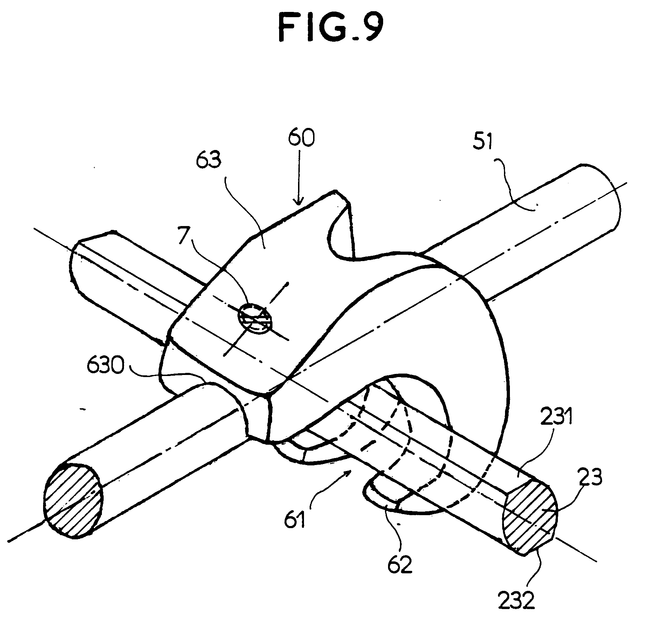

En examinant maintenant plus en détail les figures 9 à 13, on remarque que, pour assurer l'assemblage du dispositif en reliant les implants préalablement montés sur les vertèbres en procédant comme indiqué ci-dessus, il suffit, après avoir mis successivement chacune des barres 51, 52 en contact avec les étriers des implants et en maintenant ceux-ci dans leur position idéale de relier chaque étrier aux deux barres 51, 52 en utilisant les crochets 60 à fente 61 qui seraient placés tout d'abord à cheval sur leurs barres 51, 52 respectives un peu au dessus de l'étrier en maintenant la barre au contact de la cannelure semi-cylindrique 630 réalisée dans la face interne du talon 63 du crochet, puis de faire coulisser ledit talon 63 vers l'étrier de l'implant, jusqu'à mise en contact de celui-ci contre le bord interne du bec 62 du crochet 60. Il suffit alors de visser progressivement la vis 7 montée dans le talon 63 du crochet 60, pour obtenir, par basculement dudit talon 63 autour de l'arête 631 de la cannelure semi-cylindrique 630 l'emprisonnement, tout d'abord, de l'étrier derrière le bec 62 du crochet 60, puis, en poursuivant cette action, le blocage définitif en translation, en rotation et en pivotement des implants par rapport à leurs barres de liaison 51, 52, avec maintien en position idéale grâce aux méplats 231, 232 et 331, 332 réalisés sur les étriers des dits implants.

On comprend que, par ce moyen, il soit possible d'assurer aussi bien la réduction des déformations que le maintien de la réduction voulue d'un rachis.

L'efficacité tridimensionnelle du dispositif assure, en conséquence, simultanément la correction des trois composantes de la déformation scoliotique ainsi que la restitution des courbures physiologiques sagittales, la cyphose et la lordose.

## Revendications

1. Dispositif de redressement, d'étaiement et d'ostéosynthèse rachidienne à implants destinés à être fixés aux vertèbres, reliés entre eux par au moins une barre (51, 52) par l'intermédiaire d'un moyen de solidarisation amovible (60), caractérisé en ce que les implants sont constitués, pour les vertèbres dorsales, de deux crochets (21, 22 et 31, 32) reliés par un étrier (23, 33) et, pour les vertèbres lombaires, de deux anneaux reliés par un étrier, en ce que le moyen de solidarisation amovible des implants (20, 30) aux barres de liaison (51, 52) est constitué d'un crochet basculant (60) à fente (61) comportant dans le fond de la fente (61) une cannelure (630) semi-cylindrique de rayon correspondant à celui des barres (51, 52) de liaison et dans le plan médian de son talon (63) un orifice fileté (632) dans lequel est montée une vis de pression (7) permettant d'obtenir le basculement longitudinal du crochet (60) jusqu'au blocage de l'étrier des implants, et dont le bec (62) est conformé selon la section de l'étrier (23, 33) de liaison des crochets ou des anneaux des implants.

2. Dispositif selon la revendication 1, caractérisé en ce que les implants (20, 30) à crochets (21, 22 et 31, 32) sont, à crochets de même sens, à crochets (21, 22) inversés avec crochet gauche (21) vers le haut et à crochets inversés (31, 32) avec crochet droit (32) vers le haut.

3. Dispositif selon la revendication 1, caractérisé en ce que l'étrier (23, 33) de liaison des crochets (21, 22 et 31, 32) épouse la courbure des vertèbres et comporte des méplats (231, 232 et 331, 332) opposés d'immobilisation en rotation par rapport aux barres sous l'action du crochet (60) à fente (61).

4. Dispositif selon la revendication 1, caractérisé en ce que l'orifice fileté (632) dans lequel est montée la vis de pression (7) de basculement du crochet (60) à fente (61), est réalisé obliquement selon un angle d'environ 25° vers l'arrière avec un certain décalage (D) par rapport à l'aplomb du bec (62) du crochet (60).

5. Dispositif selon la revendication 1, caractérisé en ce que la base du crochet (60) à fente (61) est prolongée, du côté de l'orifice fileté (632) recevant là vis (7) de basculement et de blocage pour constituer un bras de levier destiné à accentuer l'effort de blocage de l'étrier (23, 33) de liaison des crochets ou des anneaux des implants contre les barres de jonction (51, 52).

## Claims

1. A spinal osteosynthesis device for supporting straightening and fixing the spine, comprising implants anchored on the vertabrae, at least one connecting bar (51, 52) for supporting said implants and removable elements for locking the implants onto the bars **characterized in that** the implants applied to dorsal vertebrae have two hooks (21, 22 and 31, 32) and a yoke (23, 33) between the hooks, the implants applied to lumbar vertebrae have two eyes and a yoke (23, 33) between them, the elements (20, 30) for locking the implants onto the bars (51, 52) are tilting hooks (60) each having a slot (61) and a groove (630) with a semi-cylindrical bottom, the radius of the semi-cylindrical part being-the same as that of the bars (51, 52) and a threaded bore (632) in the body 63 of the hook (60) wherein an adjusting screw (7) is arranged for tilting the hook (60) until the yoke (23, 33) connecting the hooks or eyes is locked and the claws (62) of the hooks (60) are conform with the cross-section of the yokes (23, 33).

2. The device as claimed in claim 1, **characterized in that** the implants (20, 30) are provided with hooks (21, 22 and 31, 32) bended in the same sense, hooks (21, 22) bended in opposite directions, the left hook (21 being directed upwardly and hooks 31, 32 bended in opposite directions, the right hook (32) being directed upwardly.

3. The device as claimed in claim 1, **characterized in that** the yokes (23, 33) connecting the hooks (21, 22 and 31, 32) are embracing the curvature of the vertebrae and preventing rotation with respect to the bars by opposite flatterings (231, 232 and 331, 332) and by the slotted hook 60.

4. The device as claimed in claim 1, **characterized in that** the threaded bore (632) with the adjusting screw (7) tilting the slotted hook (60) is arranged backwardly along an angle of about 25° and at a certain distance (D) from the vertical plane of the claws (62) of the hook (60).

5. The device as claimed in claim 1, **characterized in that** the base of the slotted hooks (60) is prolonged on the side of the threaded bore (632) receiving the adjusting and fixing screw (7) for providing a level to increase the locking effect between the yoke (23, 33) connecting the hooks or eyes of the implants and the bars (51, 52).

## Patentansprüche

1. Vorrichtung zum Ausrichten, Abstützen und für die Osteosynthese der Wirbelsäule mittels zum Befestigen an den Wirbeln bestimmten Einsätzen, die miteinander mit Hilfe von mindestens einer Stange (51, 52) über ein zwischenliegendes lösbares Befestigungsmittel (60) verbunden sind,
dadurch gekennzeichnet,
daß die Einsätze, für die Rückenwirbel, zwei über einen Bügel (23, 33) verbundene Haken (21, 22 und 31, 32) und, für die Lendenwirbel, zwei über einen Bügel verbundene Ringe aufweisen, daß das lösbare Befestigungsmittel der Einsätze (20, 30) an den Verbindungsstangen (51, 52) einen Kipphaken (60) mit einem Spalt (61) aufweist, der an dem Boden des Spalts (61) eine halbzylindrische Hohlkehle (630) mit einem Radius, der demjenigen der Verbindungsstangen (51, 52) entspricht, und in der Medianebene seines hinteren Endes (63) eine mit einem Innengewinde versehene Öffnung (632) aufweist, in der eine Klemmschraube (7) montiert ist, wodurch es erlaubt wird, das Kippen in Längsrichtung des Kipphakens (60) bis zum Festsetzen des Bügels der Einsätze zu erreichen, wobei das Hakenende (62) des Kipphakens dem Querschnitt des die Haken oder die Ringe der Einsätze verbindenden Bügels (23, 33) angepaßt geformt ist.

2. Vorrichtung gemäß Anspruch 1, dadurch gekennzeichnet, daß die Einsätze (20, 30) mit Haken (21, 22 und 31, 32) solche mit gleichgerichteten Haken, solche mit umgekehrt ausgerichteten Haken (21, 22) mit dem linkem Haken (21) nach oben und solche mit umgekehrt ausgerichteten Haken (31, 32) mit dem rechtem Haken (32) nach oben sind.

3. Vorrichtung gemäß Anspruch 1, dadurch gekennzeichnet, daß der die Haken (21, 22 und 31, 32) verbindende Bügel (23, 33) sich an die Krümmung der Wirbel anschmiegt und einander gegenüberstehende Abflachungen (231, 232 und 331, 332) zum rotatorischen Festsetzen relativ zu den Stäben unter Einwirkung des Hakens (60) mit Spalte (61) aufweist.

4. Vorrichtung nach Anspruch 1, dadurch gekennzeichnet, daß die mit Innengewinde versehene Öffnung (632), in der die Stellschraube (7) zum Kippen des Hakens (60) mit Spalte (61) montiert ist, mit einer bestimmten Versetzung (D) schräg nach hinten in einem Winkel von etwa 25° relativ zu der lotrechten Lage des Schnabels (62) des Hakens (60) angeordnet ist.

5. Vorrichtung gemäß Anspruch 1, dadurch gekennzeichnet, daß die Basis des Hakens (60) mit Spalte (61) an der Seite der mit Innengewinde versehenen Öffnung (632), die die Schraube (7) des Schwenkens und des Blockierens aufnimmt, verlängert ist, so daß ein Hebelarm gebildet ist, der bestimmt ist, die Feststellkraft des die Haken oder die Ringe der Einsätze verbindenden Bügels (23, 33) gegen die Verbindungsstangen (51, 52) zu verstärken.
